# EUROPEAN PATENT APPLICATION

(11) **EP 1 870 471 A1**
(43) Date of publication of application: **26.12.2007**
(21) Application number: 06076281.2
(22) Date of filing: 22.06.2006
(51) Int. Cl.: C12N 15/82, A01H 1/06

(54) **Method for generation of mutants in Plants**

(71) Applicant: Universiteit Leiden, 2312 AV Leiden (NL)
(72) Inventor: van der Zaal, Engelbertus Jacobus, 2333 VM Leiden (NL); Hooykaas, Paul Jan Jacob, 2343 RS Oegstgeest (NL)
(74) Representative: van Loon, C.J.J.

(57) **Abstract**

One aspect of the invention is to generate mutants in plants, preferably *Arabidopsis,* by transforming said plants with pools of zinc finger artificial transcription factors (ZF-ATFs) and selecting a mutant from those transformed plant with a desired phenotype.

A further aspect of the present invention is a transgenic plant harbouring a specific ZF-ATF, providing the plant with an increase in the frequency of homologous recombination (HR), more specifically wherein said ZF-ATF is likely to bind to the recognition sequence 5'-GTGGAGGCT-3'.

## Description

The invention relates to methods to improve homologous recombination in plants, more specifically by expression of zinc finger encoding sequences.

Zinc finger artificial transcription factors (ZF-ATFs), consisting of a polydactyl zinc finger (PZF) DNA binding domain linked to a protein domain which either activates or represses gene expression nearby the PZF binding site, have recently gained enormous attention (Blancafort *et al.,* 2004; Blancafort *et al.,* 2005; Eberhardy *et al.,* 2006). ZF-ATFs are generally constructed using Cys₂His₂ ZF domains, the most thoroughly characterized ZF moieties to date. Apart from being small, just 30 amino acids, Cys₂His₂ ZF domains typically bind 3 contiguous DNA bases per ZF and are highly modular. These features allow construction of more complex PZF fusion proteins, which in principle can recognize unique sites within a complex genome. For GNN, ANN and recently also CNN target sites, where N represents any of the 4 bases, the most optimal zinc finger designs have been characterized, making ZF-ATF construction possible for almost any target site (Dreier *et al.,* 2001; Dreier *et al.,* 2005; Segal *et al.,* 1999). In Arabidopsis it has been demonstrated that a ZF-ATF containing six ZF domains, designed for the endogenous APETELA (AP3) promoter, was able to specifically regulate the expression of a promoter-GUS fusion (AP3::GUS) *in planta* (Guan *et al.,* 2002). Recently, also PZF domains consisting of only three ZF domains and thus designed for a 9 bp binding site were shown to be useful for gene regulation in Arabidopsis (Holmes-Davis *et al.,* 2005; Van Eenennaam *et al.,* 2004).

Apart from being instrumental for interfering with expression levels of specific genes, ZF-ATFs can in principle also be used for a random mutagenesis in order to reveal novel phenotypes. To this end, pools consisting of large numbers of ZF-ATFs were introduced into *Escherichia coli* and yeast cells, as well as various mammalian cell lines to screen for phenotypes ranging from thermotolerance, osmotolerance, increased protein yield to drug resistance (Blancafort *et al.,* 2003; Kwon *et al.,* 2006; Lee *et al.,* 2004; Park *et al.,* 2003; Park *et al.,* 2005a; Park *et al.* 2005b). The ZF moieties used for these purposes possessed modest complexity and, as such, unique genomic target sites are unlikely. This, however, is at the same time the strength of ZF-mediated mutagenesis: with a relatively low number of ZF-ATFs, saturating coverage of the genome is assured due to the large number of potential DNA binding sites. So far, this application of pools of ZF-ATFs for mutagenesis has been restricted to the cellular level and has not been reported in multi-cellular higher eukaryotes like for instance whole *Arabidopsis* plants.
Here we describe the use of pools of three-fingered (3F) ZF-ATFs to obtain homologous recombination (HR) mutants in *Arabidopsis.* An understanding of the regulation of HR events is of great interest, not only from a pure scientific perspective, but also since HR is required for the introduction of novel traits at a precise locus via genetic engineering and controls the process of meiosis. Unfortunately, in higher eukaryotic organisms and especially in plants, almost all incoming DNA molecules that integrate into the genome do so apparently at random positions via non-homologous recombination (NHR) (Offringa *et al.,* 1990; Paszkowski *et al.,* 1988). Efficient procedures for gene modification in plants will thus require either a suppressed NHR pathway and/or a strongly activated HR pathway. Several methods to achieve this have been published in the meantime (Bundock *et al.,* EMBO Journal (1995); Bundock *et al.,* PNAS (1996); Gouka *et al.,* (1999); WO 0068404; WO 0012716; Tsukamoto *et al.,* NAR (1996) and Bundock *et al.,* 1999). Also, several mutants have been described with alterations in HR frequency, such as the *atino80*-1 mutant (Fritsch *et al.,* 2004) which shows a reduced HR frequency, and a hyperrecombogenic phenotype due to reduced expression of At*CEN2* (Molinier *et al.,* 2004). In this study, we used an *in planta* recombination assay (Swoboda *et al.,* 1994) to select for mutants up-regulated in HR after transformation of *Arabidopsis* plants with pools of ZF-ATFs. In this manner we were able to isolate a novel mutant exhibiting a dramatic increase in the frequency of HR events. Our findings demonstrated that also at the level of the multi-cellular organism ZF-ATF technology is readily applicable for identification of novel types of mutants.

Accordingly, one aspect of the invention is to generate mutants in plants, preferably *Arabidopsis,* by transforming said plants with pools of zinc finger artificial transcription factors (ZF-ATF) and selecting a mutant from those transformed plant with a desired phenotype.
A further aspect of the present invention is a transgenic plant harbouring a specific ZF-ATF, providing the plant with an increase in the frequency of homologous recombination (HR), more specifically wherein said ZF-ATF is designed to bind to the recognition sequence 5'-GTGGAGGCT-3'

### LEGENDS TO THE FIGURES

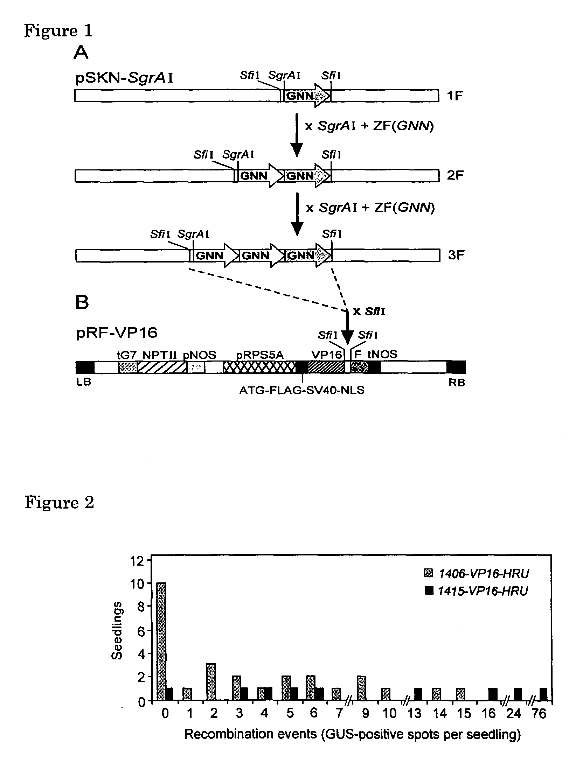

Fig. 1 Schematic representation of the construction of 3F ZF-ATF pools. A: Annealed oligonucleotide pairs encoding ZF domains were ligated in a directional manner into the *Sgr*AI site of vector pSKN-S*gr*AI, resulting in one finger (1F) and two finger (2F) constructs. Three finger (3F) pools with a complexity of 256 3Fs per pool were each formed separately by introduction of a third ZF domain into an equimolar mix of sixteen *Sgr*AI-digested 2F constructs. The resulting 3F pools were named after the identity of the first ZF cloned (shaded 'GNN' arrow). In this manner, fifteen of the possible sixteen 3F pools were constructed. B: The combined 3F sequences of each pool were isolated as *Sfi*I fragments and cloned in a directional manner into T-DNA vector pRF-VP16 for *in planta* expression of fusion proteins via the RPS5A promoter (pRPS5A). When expressed, the N-terminus of the 3F domains will be preceded by a FLAG tag (directly following the ATG translational start codon), a SV40 nuclear localization signal (SV40-NLS) and the VP16 transcriptional activator domain (VP16). A translational stop is provided after a 37 amino acid C-terminal sequence of the VirF protein of *Agrobacterium tumefaciens* (F), transcriptional top by the NOS terminator (tNOS). The *NPTII* gene is present as a plant selectable marker. LB: left border, RB: right border.

Fig. 2 Distribution of recombination frequency in individual pRF-VP16-HRU primary transformants in lines *1406* and 1415. Recombination frequency in 10 day old primary transformants were determined as number of GUS positive spots per seedling.

Fig. 3 Effect of genotoxic chemicals on HR frequency and fresh weight development in parental control *(1406)* and *VP16-HRU* expressing seedlings. A: Distribution of HR frequency per seedling in mock-treated seedlings (top) and seedlings exposed for 24 hr to bleomycin (middle) or MMS (bottom). HR events were determined 5 days after treatment as number of GUS positive spots per seedling. B: Effect of long term (2 weeks) treatment with bleomycin or MMS on fresh weight development of wt *Columbia* (Co10), *1406* and *1406-VP16-HRU* seedlings.

Fig. 4A DNA sequence coding for the *VP16-HRU* gene. B: Amino acid sequence encoded by the *VP16-HRU* gene.

Fig. 5A DNA sequence coding for the three zinc fingers. B: Amino acid sequence derived from the DNA sequence of Fig. 5A.

### DETAILED DESCRIPTION OF THE INVENTION

Although the technique using ZF-ATFs to induce dominant mutations is used at the cellular level, it was unknown whether this technique would also be useful to provide for an efficient manner of isolating phenotypes at the level of complete organisms, in particular plants, more in particular *Arabidopsis* plants. The advantage of the ZF-ATF technique is that it is a robust mutagenesis protocol without the need of generating huge numbers of transgenic organisms. The use of sub-pools of ZF-ATFs with relatively low complexity, combined with the large amount of genomic target sites for each zinc finger, has in the current invention shown to allow for an unbiased screening at almost saturating level. Assuming that all base pairs occur at equal frequency, a complete pool of 3F DNA binding domains recognizing (GNN)₃ targets will find a target site every 32 bp in a doubled stranded genome. On average, any specific 3F domain will encounter the optimal cognate 9 bp target site once within 131,000 bp (4⁹/2 bp for dsDNA). Given the size of a plant genome (in diploid plants) each specific (GNN)₃ target site occurs at least hundreds of times within the genome (e.g. on average 800 times for *Arabidopsis).* This means that, even when only a minority of the possible DNA targets will be available for 3F binding, the possibility that at least one member of a 3F ZF-ATF pool has access to a binding site at a position from which the transcriptional activity of a gene can be influenced should be quite large. However, still the possibility exists that zinc finger binding in plants does not create the same effect as in bacteria or in animal cells, since the organisation of the genome with relation to expression of genes is quite different. At the one hand, it would therefore be unsure whether ZF-ATF binding, even when it occurred, would be an efficient means also give rise to influences of transcriptional activity of neighbouring genes. At the other hand, supposing that a different organisation of the genome does not form a major obstacle, it can be envisaged that expression of frequently binding and theoretically active ZF-ATF imposes serious problems during multicellular development. In that case, one can predominantly expect a collection of monstrosities when ZF-ATF technology is used for generation of mutant collections in multicellular organisms. This would hamper screening for informative mutations and make the method as such useless. However, it appeared feasible, even by generating a relative small number of transgenic plants, to screen for mutations by using ZF-ATFs. In this respect, mutation analysis using ZF-ATFs appears more advantageous than T-DNA activation tagging (Weigel *et al.,* 2000), which in principle acts in a similar manner, because the number of independent transgenic organisms required for a saturated screening through T-DNA activation tagging depends on the genome size in a linear fashion. This effect does not show clearly with plants with short and simple genomes, but is most clear in plants with larger genomes, such as polyploids.

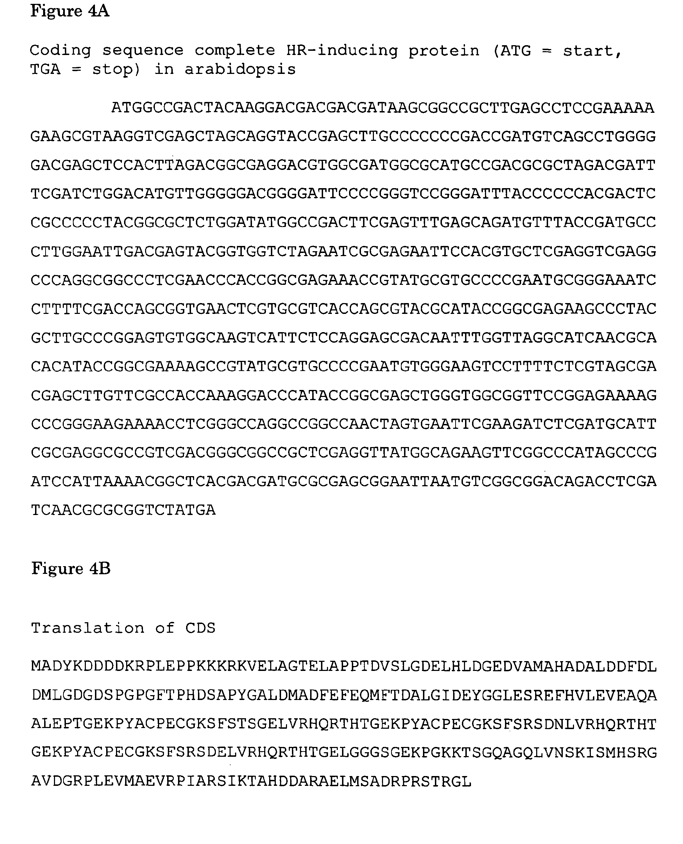

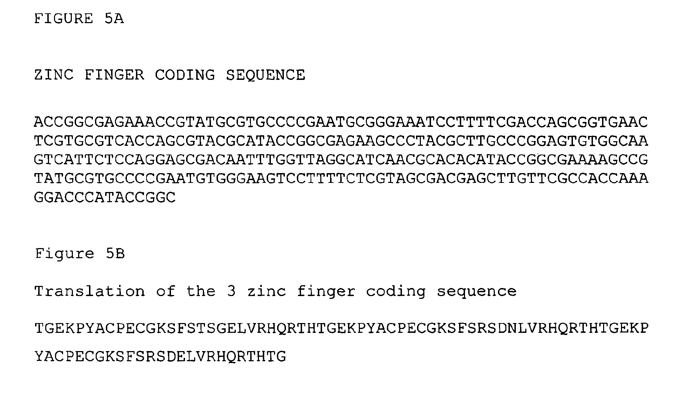

As is shown in the Experimental part, it has been possible to find an *Arabidopsis* mutant with up regulated HR frequencies. The 3F ZF-ATF gene *VP16-HRU* acts as a dominant factor, causing a 200 to 1000-fold increase in two different HR reporter lines, *1406* and 1415. These frequencies are much higher than reported previously using the same or related types of reporter loci; the most dramatic HR increase described thus fat being 36-fold for the *AtCEN2* mutant (Molinier *et al.,* 2004). The gene *VP16-HRU* codes for a VP16 activation domain fused with a 3F (three zinc fingers) moiety, which according to the current knowledge optimally recognizes the 9 bp sequence 5'-GTGGAGGCT-3', and a short C-terminal sequence derived from the VirF protein of *Agrobacterium tumefaciens.* One embodiment of this gene is represented in Fig. 4A (encoded protein in Fig. 4B), which shows the actual sequence used in the experiments. For exerting an effect on HR, it would however suffice to express the amino acid sequence providing for the three zinc fingers, such as depicted in Fig. 5B fused with effector domains of interest. Advantageously, as is done in the experiments, an additional activator, such as VP16 may be employed to enhance HR. The choice of construct and the control elements in said construct, e.g. promoter, enhancer and or the regulatory sequences that are translationally fused to the ZF domain, may further influence the specific recombination processes that are influenced by expression of the resulting 3F ZF-ATF. It can, for instance be envisaged, that a combination with other effector domains and/or other promoters can specifically influence recombination processes, e.g. the repression of meiotic recombination if a repressor domain, such as for instance the EAR domain (Ohta M, Matsui K, Hiratsu K, Shinshi H, Ohme-Takagi M. Repression domains of class II ERF transcriptional repressors share an essential motif for active repression.Plant Cell. 2001 Aug;13(8):1959-68) is used in combination with the particular 3F domain under control of a promoter that is active during meiosis (like the *DMC1* promoter [Doutriaux MP, Couteau F, Bergounioux C, White C. Isolation and characterisation of the RAD51 and DMC1 homologs from Arabidopsis thaliana. Mol Gen Genet. 1998 Feb;257(3):283-91]. Any activating or repressing domains known to the person skilled in the art would be usable in the present invention, dependant on the specifically desired effects.

As it appears that its recognition sequence is not present within the sequences of the HR reporter loci or in the flanking regions of these loci, VP16-HRU must directly or indirectly activate essential regulatory components of a HR pathway, rather than acting directly on the reporter loci. Thus, it is clear that one of the genes that leads to effects upon the HR pathway comprises a matching recognition sequence for the 3F ZF moiety of VP16-HRU within the gene itself, but more likely in the control elements controlling expression of the gene (i.e. promoter, activator and enhancer sequences). On basis of the genomic information, it is possible to define the loci which harbour suitable recognition sequences for VP16-HRU and test the corresponding genes or regulators for the involvement in the HR pathway via knock-out or overexpression studies. Genome wide transcriptional analysis of plant material expressing VP16-HRU and therefore exhibiting changes in HR compared to plant material lacking such constructs, provides a more attractive option. Those loci that are differently expressed as a direct or indirect consequence of VP16-HRU action can be identified and tested with said methods for an effect on HR. In that manner, the endogenous key gene(s) involved in HR regulation in can be identified, allowing the subsequent experimental manipulation of HR.

Another part of the invention is a plant, which is transformed with a nucleotide sequence encoding the three zinc fingers depicted in Fig. 5B. Preferably, said nucleotide sequence is the sequence depicted in Fig. 5A. However, due to the wobble in the genetic code, some triplets may be changed for others without changing the encoded protein, wherein especially the third base in a triplet may differ from the sequence as indicated in Fig. 5A. As indicated above, the presence of an activator like VP16 is advantageous for the present invention. In such a case, the nucleotide sequence to be transformed into a plant is preferably coding for the amino acid sequence of Fig. 4B. This nucleotide sequence comprises the open reading frame under control of the *pRPS5A* promoter as depicted in Fig. 1B, thus including the 3F ZF domain of VP16-HRU fused with the VP16 domain and some miscellaneous sequences. A nuclear localization domain, in this particular case from the virus SV40, provides the essential targeting of the fusion protein to the nucleus. Preferably said nucleotide sequence is the DNA sequence depicted in Fig. 4A.

A polynucleotide construct for the transformation of plants comprises a recombinant polynucleotide for expression of the three zinc finger protein sequence. As indicated above, said construct preferably comprises a nucleic acid which codes for a protein according to Fig. 5B or a functional fragment thereof. A functional fragment of said protein is defined as a protein which is highly homologous to the protein depicted in Fig. 5B and which remains functional when expressed in a plant, wherein said functionality means that the specific binding characteristics of the three zinc fingers are retained.

Highly homologous in this sense means that an amino acid sequence has a sequence identity of more than 50%, preferably more than 70%, more preferably more than 80% and most preferably more than 90% with the above mentioned sequence.

For calculation of percentage identity the BLAST algorithm can be used (Altschul et al., 1997 Nucl. Acids Res. 25:3389-3402) using default parameters or, alternatively, the GAP algorithm (Needleman and Wunsch, 1970 J. Mol. Biol. 48:443-453), using default parameters, which both are included in the Wisconsin Genetics Software Package, Genetics Computer Group (GCG), 575 Science, Madison, Wisconsin, USA. BLAST searches assume that proteins can be modeled as random sequences. However, many real proteins comprise regions of nonrandom sequences which may be homopolymeric tracts, short-period repeats, or regions enriched in one or more amino acids. Such low-complexity regions may be aligned between unrelated proteins even though other regions of the protein are entirely dissimilar. A number of low-complexity filter programs can be employed to reduce such low-complexity alignments. For example, the SEG (Wooten and Federhen, 1993 Comput. Chem. 17:149-163) and XNU (Claverie and States, 1993 Comput. Chem. 17:191-201) low-complexity filters can be employed alone or in combination.

As used herein, 'sequence identity' or 'identity' in the context of two protein sequences (or nucleotide sequences) includes reference to the residues in the two sequences which are the same when aligned for maximum correspondence over a specified comparison window. When percentage of sequence identity is used in reference to proteins it is recognized that residue positions which are not identical often differ by conservative amino acid substitutions, where amino acids are substituted for other amino acid residues with similar chemical properties (e.g. charge or hydrophobicity) and therefore do not change the functional properties of the molecule. Where sequences differ in conservative substitutions, the percentage sequence identity may be adjusted upwards to correct for the conservative nature of the substitutions. Sequences, which differ by such conservative substitutions are said to have 'sequence similarity' or 'similarity'. Means for making these adjustments are well known to persons skilled in the art. Typically this involves scoring a conservative substitution as a partial rather than a full mismatch, thereby increasing the percentage sequence identity. Thus, for example, where an identical amino acid is given a score of 1 and a non-conservative substitution is give a score of zero, a conservative substitution is given a score between 0 and 1. The scoring of conservative substitutions is calculated, e.g. according to the algorithm of Meyers and Miller (Computer Applic. Biol. Sci. 4:11-17, 1988).

The polynucleotide construct of the present invention is preferable constructed such that it comprises at least and in operable linkage a first promoter that is functional in plants, a nucleotide sequence encoding a protein with the three zinc fingers, and a terminator. Preferably said construct also comprises a sequence coding for an activator protein, such as VP16. Optionally the polynucleotide may comprise a gene sequence encoding a selectable or screenable trait operably linked to regulatory sequences for expression.

Preferably a viral promoter, such as the promoter from cassava vein mosaic virus (CVMV) or the promoter from cauliflower mosaic virus (CMV). However, any promoter that provides for constitutional expression (such as the 35S or the enhanced 35S promoter) may be used. As indicated above, for specific effects in specific tissues or developmental stages, tissue-specific or stage-specific promoters can be used, such as a meiose-specific promoter (DCM1 promoter,: Doutriaux MP, Couteau F, Bergounioux C, White C. Isolation and characterisation of the RAD51 and DMC1 homologs from Arabidopsis thaliana. Mol Gen Genet. 1998 Feb;257(3):283-91or inducible systems, such as Tamoxifen inducible promoters (Friml J et al. A PINOID-dependent binary switch in apical-basal PIN polar targeting directs auxin efflux. Science. 2004 Oct 29;306(5697):862-5.]. Inducible or gene switch promoters can be used, which need not to be tissue specific, as long as they show expression in the tissue or developmental stage, where expression is desired. The advantage of such inducible promoters is that they are silent when not induced, and thus the plants can develop and grow unhindered by the transgenic construct.. Inducible promoters include any promoter capable of increasing the amount of gene product produced by a given gene in response to exposure to an inducer. In the absence of an inducer, the DNA sequence will not be transcribed. Typically, the factor that binds specifically to an inducible promoter to activate transcription is present in an inactive form which is then directly or indirectly converted to the active form by the inducer. The inducer may be a chemical agent such as a protein, metabolite (sugar, alcohol, *etc.),* a growth regulator, herbicide, or a phenolic compound or a physiological stress imposed directly by heat, salt, wounding, toxic elements *etc.,* or indirectly through the action of a pathogen or disease agent such as a virus. A plant cell containing an inducible promoter may be exposed to an inducer by externally applying the inducer to the cell such as by spraying, watering, heating, or similar methods. Inducible promoters are known to those familiar with the art and several exist that could conceivably be used to drive expression of the 3F moiety. Inducible promoters suitable for use in accordance with the present invention include, but are not limited to, the heat shock promoter, promoters inducible by the mammalian steroid receptor system and any chemically inducible promoter. Examples of inducible promoters include the inducible 70 kD heat shock promoter of *Drosophila melanogaster* (Wing et al 1989, Mol Gen Genet, 219: 9-16) and the alcohol dehydrogenase promoter which is induced by ethanol (Freeling, M. et al., Ann. Rev. Genet. 19, 297-323; Nagao, R.T. et al., in: Miflin, B.J. (ed.) Oxford Surveys of Plant Molecular and Cell Biology, Vol. 3., pp. 384-438, Oxford Univ. Press, 1986). A promoter that is inducible by a simple chemical is particularly useful. Such simple or common chemicals are used in the induction of so-called gene switch promoters. Examples of gene switch promoters include the alcA/alcR gene switch promoter as described in published International Patent Application No. WO 93/21334; the GST promoter, as described in published International Patent Application Nos. WO 90/08826 and WO 93/031294; and the ecdysone switch system as described in published International Patent Application No. WO 96/37609. In such switch systems, the timing of gene expression is controlled by application of an external chemical. The switch chemical may be applied as a spray or vapor to all or part of the transgenic plant or as a root drench. Examples of suitable switch chemicals are provided in the above references describing switch promoter systems. The external chemical stimulus is preferably an agriculturally acceptable chemical, the use of which is compatible with agricultural practice and is not detrimental to plants or mammals. Inducible switch promoter systems preferably include one or two component systems; nevertheless, systems comprising more than two components are encompassed by the present invention. The switch system may be driven by a constitutive promoter or by a tissue or organ-specific promoter, whereby the target gene is only switched on in a target tissue or organ. The alcA/alcR switch promoter system is particularly preferred. In the alcA/alcR promoter switch system, the preferred chemical inducer is ethanol, in either liquid or vapour form. One of the main advantages of the use of ethanol is that small quantities of ethanol generate high levels of expression. The alcA/alcR inducible promoter system is a two-component system involving DNA sequences coding for the alcA promoter and the alcR protein, the expression of which is placed under the control of desired promoters. The alcR protein activates the alcA promoter in the presence of an inducer and any gene under the control of the alcA promoter (in this case the *GGL* gene), will therefore be expressed only in the presence of that inducer. With such a system the activity of the gene construct can be limited both by place and by time. Other gene-switch systems and/or inducible promoters are known in the art and would also be equally applicable.

The recombinant gene constructs may be inserted into a vector, which may be commercially available, suitable for transforming into plants and suitable for expression of the gene product in the transformed cells. Preferably used are binary vectors (such as pMOG22, known from Goddijn, O.J.M. et al., 1993, Plant J, 4:863-873) which are useful for plant transformation using *Agrobacterium.*

In principle any transformation method may be used to introduce chimeric DNA according to the invention into a suitable ancestor cell. Methods may suitably be selected from the calcium/polyethylene glycol method for protoplasts, electroporation of protoplasts, microinjection into plant material, (DNA or RNA-coated) particle bombardment of various plant material, infection with (non-integrative) viruses, in planta *Agrobacterium tumefaciens* mediated gene transfer by infiltration of adult plants or transformation of mature pollen or microspores (EP 0 301 316) and the like. A preferred method according to the invention comprises *Agrobacterium-*mediated DNA transfer. Especially preferred is the use of the so-called binary vector technology as disclosed in EP 0 120 516 and U.S. Patent 4,940,838.

A method for production of a transgenic plant or plant part according to the invention may comprise the step of selecting transformed plants or plant parts. Generally after transformation, plant cells or cell groupings are selected for the transfer with the polynucleotide construct comprising the DNA-sequence with the genes encoding the various enzymes or blocking mechanisms according to the invention, following by steps know to the skilled person by which the transformed material is regenerated into a whole plant and evaluating the transformed plant for the overproduction of glycerol.

Selectable markers, which may be included as a part of the introduced recombinant DNA, are used to select transformed cells (those containing recombinant DNA) over untransformed cells. Examples of suitable markers include genes that provide antibiotic or herbicide resistance. Cells containing the recombinant DNA are capable of surviving in the presence of antibiotic or herbicide concentrations that kill untransformed cells. Examples of selectable marker genes include the bar gene which provides resistance to the herbicide Basta; the nptII gene which confers kanamycin resistance; the hpt gene which confers hygromycin resistance; and the cah gene which gives resistance to cyanamid. An entire plant can be generated from a single transformed plant cell through cell culturing techniques known to those skilled in the art.

A process for obtaining a transgenic plant according to the invention may in an alternative embodiment comprise introducing a vector according to the invention into an ancestor plant, and then producing said transgenic plant from said ancestor plant.

Yet another alternative embodiment for obtaining a transgenic plant according to the invention may comprise introducing a polynucleotide construct according to the invention into a suitable vector for transforming a plant part to produce a transformed plant part, and then regenerating said transgenic plant from said transformed plant part.

Following DNA transfer and regeneration, putatively transformed plants may be evaluated, for instance using Southern analysis, for the presence of the recombinant DNA according to the invention, copy number and/or genomic organization. In addition, or alternatively, expression levels of the newly introduced DNA may be undertaken, using Northern and/or Western analysis, techniques well known to persons having ordinary skill in the art. Further, as is exemplified in the Example, analyses can be performed which would show an increased frequency of homologous recombination, such as the GUS assay as essentially described in Swoboda *et al.,* 1994..

### EXAMPLES

### Experimental procedures

### Construction of plant expression vector pRF-VP16

Plant expression vector pGPTV-KAN (Becker *et al.,* 1992) was modified in order to obtain pRF-VP16. Briefly, *Not*I and *Sfi*I sites within the vector backbone were removed via sequential digestion and treatment with Klenow enzyme and T4 DNA polymerase, respectively. These modifications did not hamper the frequency of plant transformation. The promoterless GUS coding sequence was replaced by a 1.7 kb *Xma*I*-Sac*I fragment containing the *RPS5A* promoter (Weijers *et al.,* 2001), after which the plasmid was digested with SacI followed by insertion of a sequence providing the vector with an ATG translational start codon, a FLAG tag, a SV40 nuclear localization signal and *Not*I and XhoI sites. A XhoI-SacI fragment encoding the 37 C-terminal amino acids of the VirF protein of an octopine strain of *Agrobacterium tumefaciens* was added to the vector. Although this domain was of no further specific relevance for this study, the DNA fragment provided a translational stop codon for the fusion proteins produced *in planta.* The unique *Not*I site was used for introduction of the VP16 transcriptional activation domain (Sadowski *et al.,* 1988) and *SfiI* sites for directional cloning of zinc finger domains. The most relevant features of the resulting plant expression plasmid pRF-VP16 are shown in Figure 1B. Sequence available upon request.

### Construction of 3F ZF-ATF pools

Zinc finger modules designed for 5' GNN 3' or 5'(GNN)₂ 3' binding sites were constructed in pSKN-S*gr*AI as described previously (Neuteboom *et al.,* 2006), using annealed oligonucleotide pairs each encoding an optimal zinc finger sequence as established by (Segal *et al.,* 1999). Once a sequence-verified series of constructs encoding two-finger (2F) proteins sharing the C-terminal finger was established, the third finger was added in a controlled manner (Figure 1A). For each 2F series, the sixteen 2F constructs were grown overnight in LC medium containing carbenicillin at 100mg/l for plasmid selection and glucose (20 mM) in order to repress any untimely expression of the ZF proteins via the *lac* promoter. A larger volume (100 ml) of the same medium was subsequently inoculated with equal amounts of each of the sixteen bacterial strains belonging to the series (corresponding to 0.5 ml of bacterial culture with OD 1.0 at 600 nm) and grown for an additional 4 hours. Plasmid DNA was isolated, digested with SgrAI and subdivided for 16 separate ligation reactions with one specific ZF-encoding oligonucleotide pair present at 1000-fold molar excess relative to vector molecules. After ligation and heat inactivation of the enzyme, the reactions were pooled and linear plasmid molecules containing additional ZF oligonucleotides at each end were isolated from gel. Upon a denaturation-renaturation procedure as described previously (Neuteboom *et al.,* 2006) circularized annealed plasmid DNA was transformed to *E. coli* DH5α to obtain the 3F pool. Each pool consisted of at least 2000 independent colonies to ensure that complexity of 256 different 3F modules per pool was maintained. Colonies belonging to a pool were scraped from solid medium and grown together for an additional 5-6 hours after which the 3F-containing plasmids were isolated. The plasmid pool was digested with *SfiI* to obtain the 325 bp fragments containing 3F sequences which were subsequently ligated into SfiI-digested plant expression vector pRF-VP16 and transformed to *E. coli* DH5α. pRF-VP16 3F pools typically consisted of at least 2000 independent colonies with more than 90% recombinant plasmids. All colonies were scraped from plates and grown briefly in LC under kanamycin selection. The resulting pools were each mobilized to *Agrobacterium tumefaciens* strain AGL1 (Lazo *et al.,* 1991) via triparental mating (Ditta *et al.,* 1980). The resulting masses of *Agrobacterium* colonies were scraped from selection plates (kanamycin, rifampicin, and carbenicillin at 100, 10, and 75 mg per liter, respectively). After briefly growing pools at 28°C in selective liquid medium, 1 ml aliquots of *Agrobacterium* cultures were frozen in bactopeptone medium with 17% glycerol and stored at -80°C for starting bacterial cultures to be used for plant transformation.

### Plant transformation and homologous recombination assay

Plants of line *1406* (Gherbi *et al.,* 2001) were transformed with each of the pRF-VP16-3F pools by floral dip (Clough and Bent 1998; Lazo *et al.,* 1991) and primary transformants (T1 seedlings) were selected on MA medium (Masson and Paszkowski 1992) lacking sucrose and containing kanamycin, timentin and nystatin (50, 100 and 100 mg per liter, respectively). Ten day old primary transformants were stained for GUS activity in order to reveal HR events as GUS-positive spots or sectors essentially as described (Swoboda *et al.,* 1994) but including treatment with 95% acetone for 1 hr at -20 °C followed by three rinses with phosphate buffer (pH 7.2), 0.5 mM K₃fe(CN)₆, 0.5 mM K₄Fe(CN)₆, prior to a 14-16 hr incubation in 1 mg/ml X-gluc in the same buffer. Recombination events were determined as number of spots per seedling.

### DNA isolation and PCR analysis for identification of ZF-ATFs from T1 mutants

DNA was isolated from single GUS-stained T1 seedlings essentially as described (Murray and Thompson 1980) and dissolved in a total volume of 20 µl 1/4 TE. For each PCR-mediated rescue of the ZF sequence, 2 µl of the DNA sample was used in a PCR analysis with primers pRF-uni-fw (5'-GAAGCGTAAGGTCGAGC-3') and pRF-2pol-rev (5'-CTCGCGAATGCATCGAG-3'), amplifying a 676 bp PCR product containing the sequence encoding the 3F domains. PCR was performed in a total volume of 25 µl with 0.4 µM of each primer, 0.4 µM dNTPs and 1.25 units *Pfu*Turbo® DNA polymerase (Stratagene). Part of the PCR product was analyzed by gel electrophoresis; the remaining part was digested with *SfiI* for cloning into *Sfi*I-digested pSKN-S*gr*AI. The 3F sequence was determined after sequencing with primer M13R (5'-CAGGAAACAGCTATGACCATGA-3').

### Treatment with genotoxic chemicals

All treatments were performed in a growth cabinet at 21 °C with 16 hr light per 24 hr and under continuous gentle shaking when seedlings were incubated in liquid medium. Seeds were sown on solidified 1/2 MS medium. Five days after germination, groups of three seedlings were transferred to each well of six-wells plates (Greiner), each well containing 4 ml liquid 1/2 MS medium with or without the genotoxic agents bleomycin (0.125 mg/l) or MMS (0.007%). For the recombination assay, seedlings were treated for 24 hrs and were further incubated for 5 days in liquid medium without genotoxic agents prior to GUS staining. To monitor developmental effects, treatment of seedlings was continued for two weeks. After this period, fresh weight reduction compared to controls was determined by weighing the seedlings in batches of 18 seedlings in triplicate.

### Results

### Construction of 3F pools for plant transformation

A schematic overview of the procedure to obtain 3F ZF-ATF pools is given in Figure 1. After cloning the first 16 oligomers encoding GNN-recognizing ZF moieties into pSKN-*Sgr*AI, we invested in the systematical cloning and sequencing of all possible two fingered (2F) constructs (for details regarding cloning procedure and oligomer sequences, see supplementary material in Neuteboom *et al*., 2006). In this manner 256 2F-encoding plasmids were obtained, divided in 16 pools of 16 members, each pool labeled according to the first ZF domain cloned. As substrate for the addition of the third consecutive ZF, we used restriction enzyme digested mixtures of equimolar amounts of plasmids belonging to a pool. This protocol, although more elaborate than random multimerization and shot-gun cloning of ZF moieties, was chosen for several reasons. First of all, a better control of successful 3F-pool generation is ensured. Secondly, having the total collection of 3F containing plasmids in several non-overlapping pools of lower complexity offers the possibility to screen each of the sixteen 256-member 3F pools to different extent. This is of great advantage when, instead of screening at the cellular level, mutant phenotypes have to be selected at the level of the complete organism and handling of large numbers of transgenic individuals in a single experiment is mostly not feasible. Subdividing the larger pool of potentially mutagenic transcription factors allows a much easier recovery of interesting mutants in case an initial molecular analysis fails. For fifteen out of the possible sixteen different 3F pools, the construction was successful. For unknown reasons, the 2F clones designed for 5'-GAA-GNN-3' binding sites frequently possessed errors and as these 2F clones are needed for preparation of the GAA 3F pool, this pool was not further developed. All other libraries were finally introduced as *SfiI* fragments into the wide host range vector pRF-VP16 (Figure 1B). Once cloned within this vector, the 3F moieties were fused with an N-terminal VP16 domain, preceded by a FLAG-tag and a SV40 nuclear localization domain and linked to the *RPS5A* promoter, which normally drives expression of the ribosomal protein S5A in *Arabidopsis* (Weijers *et al.,* 2001). The choice for the *RPS5A* promoter resides in the extremely early onset of its activity, already in the zygotic stage of embryo formation, as well as its continued expression in meristematic tissues of plants. Such an expression pattern ensures that all cells present in a transformed seedling have experienced a period during which the transgene is expressed, also when the transgenic seedling directly stems from the initial transformation by the floral dip procedure. In differentiated older cells, *RPS5A* activity sharply declines (Weijers *et al.,* 2001), which avoids a prolonged expression of a transgene that might be detrimental for seedling survival and would therefore diminish the chance for successful characterization of the 3F sequence which led to a phenotype of interest.

### Screening for Arabidopsis mutants with increased homologous recombination frequencies

To monitor HR frequencies in *Arabidopsis* seedlings, we have used target line *1406* (Gherbi *et al.,* 2001), containing a HR substrate in the form of an interrupted GUS reporter gene, which can be restored after an HR event within a direct repeat of the GUS coding sequence (Swoboda *et al*., 1994). Individual recombination events can be visualized as blue spots or sectors upon histochemical staining of seedlings. Per separate pool of pRF-VP16-3F plasmids present in *Agrobacterium,* a practically realistic compromise for the number of transgenic seedlings to be generated and screened, had to be defined. With each of the fifteen pRF-VP16-3F pools having a theoretical complexity of 256 different members, screening about all (>95%) constituting *VP16:3F* encoding genes for their potential to enhance HR would require the analysis of more than 750 primary transformants for each of these pools (N= In (1-0.95)/ln (1-1/library size). As indicated in Table 1, the average number of transformants screened per pool, was around 400, which corresponds to a fair chance of about 80% for any member of the pool to be represented in a transgenic seedling.

Although we observed a variety of morphological aberrations within the collection of transformed seedlings (not shown), we focused on those seedlings that exhibited more somatic HR events than the parental line and the majority of transgenics. With a spontaneous HR frequency in line *1406* of about 0.012 events per 10 day old seedling, those transgenic seedlings with three or more HR events were considered to be candidates for *VP16:3F*-induced HR mutants. Within a total of 6400 transgenic seedlings, three met this requirement; two seedlings with three spots in pools GTC and GCA and a seedling with eight spots in pool GTG (Table 1).

### Identification of ZF-ATFs in transgenic seedlings and verification of HR-inducing potential

During preliminary experiments, several GUS staining protocols were tested for accurate determination of somatic HR events while at the same time being compatible with further molecular analysis of 3F sequences. The GUS staining method involving acetone pretreatment (see Experimental procedures) provided excellent penetration of substrate throughout the seedling and led to sharply stained cells. DNA extracted from these stained seedlings was in most cases still suitable for PCR-analysis of the 3F region of the transgene. From the seedling with eight somatic HR events, a PCR product of correct size was readily obtained, but not from the two plants with three recombination events. Whether this reflects imperfectness of the DNA recovery protocol or a true absence of an intact transgene in the other two putative mutants remains uncertain. A vital staining procedure would have allowed the generation of more biomass from putative mutants, but such procedures proved to be too ineffective to reveal single stained cells.

Sequence analysis of the PCR product obtained from the seedling with eight HR events verified the presence and identity of a 3F domain, which was subsequently recloned as a S*f*iI fragment into pRF-VP16 and introduced into *Agrobacterium.* According to the GNN recognition code (Segal *et al.,* 1999) the most optimal binding site for this 3F should be 5'-GTGGAGGCT-3' (for oligomer sequences, see Neuteboom *et al.,* 2006). Most transgenic seedlings obtained with the resulting *Agrobacterium* strain again possessed a high number of HR events, proving that the particular VP16-3F factor was the causal agent of the original phenotype. From the 27 primary transformants that were stained for GUS activity, one plant exhibited one event, while at least two events were present in 16 out of the total 27 stained seedlings, ranging from 2 to 15 HR events per seedling (Figure 2). With the average number of 3.8 HR events per seedling within the group of 27 primary transformants, the mean HR frequency after transformation with this VP16-3F factor increased approximately 300-fold, compared to the parental line, which had a HR frequency of 0.012 spots per seedling. For some individual primary transformants, those with ten or more GUS positive spots, the HR frequency apparently increased by a factor 1000 or more. The HR-inducing ZF-ATF was designated VP16-HRU and the resulting up-regulated HR phenotype in transgenic plants was designated HRU.

### The effect of VP16-HRU is independent of the HR reporter locus

To rule out that the HRU phenotype observed in reporter line *1406* after transformation with *VP16-HRU* is dependent on the precise genomic location of the reporter gene and/or on the layout of the reporter locus itself, this construct was also used to transform an independent second target line for intra-chromosomal homologous recombination. In this line *1415* (Gherbi *et al.,* 2001), the substrate for homologous recombination is composed of an interrupted GUS reporter gene as well, but arranged as an inverted repeat. Although the frequency of spontaneous recombination in this line is higher than in line *1406* (0.083 events per seedling for line 1415 versus 0.012 events per seedling for line *1406* under our conditions), the effect of *VP16-HRU* was indisputable. From nine randomly picked primary transformed seedlings, only one did not reveal any GUS positive spots while for the remaining seedlings HR frequencies as high as 76 events per 10 day old seedling were observed (Figure 2). On average, 16.3 recombination events per seedling were seen in this set of nine, indicating that the mean HR frequency had risen about 200-fold after transformation with pRF-VP16-HRU, a value comparable to that observed in line *1406.* Therefore, we can conclude that the increase in HR frequency is independent of the precise location of the reporter locus and is seen both with a direct and indirect GUS repeat. Morphological aberrancies were not observed in the seedlings with the HRU phenotype. Later in development, HRU plants did have relatively long and somewhat narrower leaves than the parental line and flowering time was slightly delayed. HRU plants were fertile and produced a normal amount of seeds.

Several T1 HRU mutants in background *1406* and *1415* were allowed to self pollinate. T2 populations showing a 3:1 segregation of the kanamycin resistance gene, indicating a single transgenic locus, were maintained to obtain homozygous T3 lines. In both types of lines, designated *1406-VP16-HRU* and *1415- VP16-HRU,* the HRU phenotype remained stable up to the T3 generation (data not shown).

### VP16-HRU expression leads to increased tolerance to the genotoxic agent bleomycin

Although it was clearly demonstrated that *VP16-HRU* triggers a higher recombination frequency in *Arabidopsis* seedlings in a dominant fashion, the molecular mechanism underlying the HRU phenotype is not immediately evident. On one hand, HRU seedlings might posses a rather directly activated HR pathway and as such be subjected to an enhanced frequency of recombination events. On the other hand, the presence of *VP16-HRU* might induce a rather general kind of genotoxic stress which triggers DNA repair and thereby also HR events, but then HR is activated in an indirect manner. An experiment in which plants are treated with DNA damaging agents can in principle discriminate between these possibilities. Bleomycin is known to cause double strand breaks (DSBs) in genomic DNA (Charles and Povirk 1998; Favaudon 1982; Menke *et al.,* 2001; Norskov-Lauritsen *et al.,* 1990), which are repaired either by HR or NHR. If in HRU plants the HR pathway is constitutively up-regulated prior to inflicting extra DSBs, the HRU plants will most likely be more tolerant to bleomycin treatment than the parental line, at least as far as the already up-regulated HR pathway can repair the sudden increase in DSBs. Alternatively, if HRU plants are already experiencing genotoxic stress, a further increase of this stress by bleomycin should more readily cause severe problems. In the latter case, plants are expected to be hypersensitive to bleomycin.

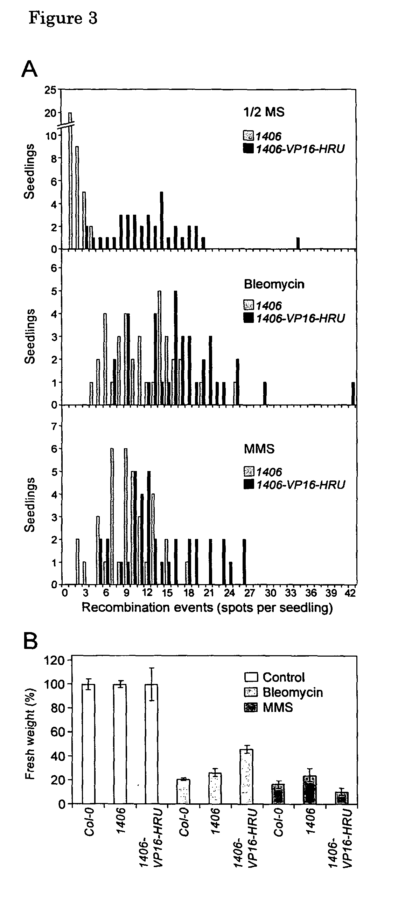

As shown in Figure 3A, a 24 hr bleomycin treatment led to a 16.5-fold induction of HR frequency in the parental line 1406, which is comparable with published data (Molinier *et al.,* 2005). HR frequency in HRU seedlings rose from a basal 11.3 events per seedling to 17.1 after bleomycin treatment, an increase of about 1.5-fold (50%). Remarkably, the basal frequency of HR events in HRU seedlings was virtually identical to the frequency observed in bleomycin-treated *1406* seedlings (11.4 HR events per seedling). In order to assess different levels of bleomycin resistance, the fresh weight of seedlings was determined after two weeks of continuous treatment. Compared with wild-type *Columbia* seedlings and the parental line *1406,* seedlings expressing *VP16-HRU* gained more fresh weight in the presence of bleomycin, thus demonstrating increased tolerance to this genotoxic compound (Figure 3B).

In addition to bleomycin, we also investigated the effect of long and short term treatments with the alkylating compound methyl methanesulfonate MMS. Genotoxic stress caused by DNA alkylation is mainly repaired via the nucleotide excision repair (NER) pathway (Lundin *et al.,* 2005). Short term MMS treatment induced the frequency of HR events per seedling of line *1406* 11.7-fold, corresponding to 8.1 events per seedling, while for HRU seedlings the frequency increased only by about 15% to 13.0 events per seedling. When analyzed in terms of fresh weight development after two weeks of treatment, HRU plants were slightly less resistant than controls (Figure 3B).

**Table 1**

| Homologous recombination events in 10-day old seedlings of *Arabidopsis* line *1406,* transformed with the different pRF-VP16-3F pools. | | | |
|---|---|---|---|
| | Seedlings with spots | | |
| Pool^{a} name and number | Number of spots | | |
| of seedlings | 1 | 2 | ≥3^{b} |
| GGG 402 | | | |
| GGA 405 | | | |
| GGT 382 | 3 | | |
| GGC 459 | | | |
| GAG 440 | 6 | | |
| GAA ND | | | |
| GAT 72 | 2 | | |
| GAC 359 | 3 | | |
| GTG 686 | 2 | 1 | 1 (8) |
| GTA 553 | 5 | | |
| GTT 384 | | | |
| GTC 634 | 10 | | 1 (3) |
| GCG 143 | 6 | | |
| GCA 415 | 1 | | 1 (3) |
| GCT 701 | 2 | | |
| GCC 365 | | | |
| **Total 6400** | **40** | **1** | **3** |
| --- 167 (*1406*)*^{c}* 2 | | | |

| | | | |
|---|---|---|---|
| a: Pools are named after the first cloned ZF domain. b: Number of spots between brackets c: Untransformed seedlings of line *1406* | | | |

### References

Bagherieh-Najjar, M.B., de Vries, O.M., Hille, J. and Dijkwel, P.P. (2005) Arabidopsis RecQI4A suppresses homologous recombination and modulates DNA damage responses. Plant J. 43:789-798.
Becker, D., Kemper, E., Schell, J. and Masterson, R. (1992) New plant binary vectors with selectable markers located proximal to the left T-DNA border. Plant Mol. Biol. 20:1195-1197.
Blancafort, P., Chen, E.I., Gonzalez, B., Bergquist, S., Zijlstra, A., Guthy, D., Brachat, A., Brakenhoff, R.H., Quigley, J.P., Erdmann, D. and Barbas, C.F. (2005) Genetic reprogramming of tumor cells by zinc finger transcription factors. Proc. Natl. Acad. Sci. U.S.A. 102:11716-11721.
Blancafort, P., Magnenat, L. and Barbas, C.F. (2003) Scanning the human genome with combinatorial transcription factor libraries. Nat. Biotechnol. 21:269-274. Blancafort, P., Segal, D.J. and Barbas, C.F. (2004) Designing transcription factor architectures for drug discovery. Mol. Pharmacol. 66:1361-1371.
Charles, K. and Povirk, L.F. (1998) Action of bleomycin on structural mimics of intermediates in DNA double-strand cleavage. Chem. Res. Toxicol. 11:1580-1585. Clough, S.J. and Bent, A.F. (1998) Floral dip: a simplified method for Agrobacterium-mediated transformation of Arabidopsis thaliana. Plant J. 16:735-743. Ditta, G., Stanfield, S., Corbin, D. and Helinski, D.R. (1980) Broad host range DNA cloning system for gram-negative bacteria: construction of a gene bank of Rhizobium meliloti. Proc. Natl. Acad. Sci. U.S.A. 77:7347-7351.
Dreier, B., Beerli, R.R., Segal, D.J., Flippin, J.D. and Barbas, C.F. (2001) Development of zinc finger domains for recognition of the 5'-ANN-3' family of DNA sequences and their use in the construction of artificial transcription factors. J. Biol. Chem. 276:29466-29478.
Dreier, B., Fuller, R.P., Segal, D.J., Lund, C.V., Blancafort, P., Huber, A., Koksch, B. and Barbas, C.F. (2005) Development of zinc finger domains for recognition of the 5'-CNN-3' family DNA sequences and their use in the construction of artificial transcription factors. J. Biol. Chem. 280:35588-35597.
Dubest, S., Gallego, M.E. and White, C.I. (2002) Role of the AtRad1p endonuclease in homologous recombination in plants. EMBO Rep. 3:1049-1054.
Dubest, S., Gallego, M.E., and White, C.I. (2004) Roles of the AtErcc1 protein in recombination. Plant J. 39:334-342.
Eberhardy, S.R., Goncalves, J., Coelho, S., Segal, D.J., Berkhout, B. and Barbas, C.F. (2006) Inhibition of human immunodeficiency virus type 1 replication with artificial transcription factors targeting the highly conserved primer-binding site. J. Virol. 80:2873-2883.
Favaudon, V. (1982) On the mechanism of reductive activation in the mode of action of some anticancer drugs. Biochimie 64:457-475.
Fritsch, O., Benvenuto, G., Bowler, C., Molinier, J. and Hohn, B. (2004) The INO80 protein controls homologous recombination in Arabidopsis thaliana. Mol Cell 16:479-85.
Gherbi, H., Gallego, M.E., Jalut, N., Lucht, J.M., Hohn, B. and White, C.I. (2001) Homologous recombination in planta is stimulated in the absence of Rad50. EMBO Rep. 2:287-291.
Guan, X., Stege, J., Kim, M., Dahmani, Z., Fan, N., Heifetz, P., Barbas, C.F. and Briggs, S.P. (2002) Heritable endogenous gene regulation in plants with designed polydactyl zinc finger transcription factors. Proc. Natl. Acad. Sci. U.S.A. 99:13296-13301.
Heitzeberg, F., Chen, I.P., Hartung, F., Orel, N., Angelis, K.J. and Puchta, H. (2004) The Rad17 homologue of Arabidopsis is involved in the regulation of DNA damage repair and homologous recombination. Plant J. 38: 954-968
Holmes-Davis, R., Li, G., Jamieson, A.C., Rebar, E.J., Liu, Q., Kong, Y., Case, C.C. and Gregory, P.D. (2005) Gene regulation in planta by plant-derived engineered zinc finger protein transcription factors. Plant Mol. Biol. 57:411-423.
Kwon, R.J., Kim, S., Lee, S.I., Hwang, S.J., Lee, G., Kim, J.S. and Seol, W. (2006) Artificial transcription factors increase production of recombinant antibodies in Chinese hamster ovary cells. Biotechnol. Lett. 28:9-15.
Lazo, G.R., Stein, P.A. and Ludwig, R.A. (1991) A DNA transformation-competent Arabidopsis genomic library in Agrobacterium. Biotechnology (N. Y.) 9:963-967.
Lebel, E.G., Masson, J., Bogucki, A. and Paszkowski, J. (1993) Stress-induced intrachromosomal recombination in plant somatic cells. Proc. Natl. Acad. Sci. U.S.A 90:422-426.
Lee, D.K., Kim, Y.H., Kim, J.S. and Seol, W. (2004) Induction and characterization of taxol-resistance phenotypes with a transiently expressed artificial transcriptional activator library. Nucleic Acids Res. 32:e116.
Lundin, C., North, M., Erixon, K., Walters, K., Jenssen, D., Goldman, A.S. and Helleday, T. (2005) Methyl methanesulfonate (MMS) produces heat-labile DNA damage but no detectable in vivo DNA double-strand breaks. Nucleic Acids Res. 33:3799-3811.
Masson, J. and Paszkowski, J. (1992) The culture response of Arabidopsis-thaliana protoplasts is determined by the growth-conditions of donor plants. Plant J. 2:829-833.
Masson, J.E., King, P.J. and Paszkowski, J. (1997) Mutants of Arabidopsis thaliana hypersensitive to DNA-damaging treatments. Genetics 146:401-407. Masson,J.E. and Paszkowski,J. (1997) Arabidopsis thaliana mutants altered in homologous recombination. Proc. Natl. Acad. Sci. U.S.A. 94:11731-11735.
Mengiste, T., Revenkova, E., Bechtold, N. and Paszkowski, J. (1999) An SMC-like protein is required for efficient homologous recombination in Arabidopsis. EMBO J. 18:4505-4512.
Menke, M., Chen, I., Angelis, K.J. and Schubert, I. (2001) DNA damage and repair in Arabidopsis thaliana as measured by the comet assay after treatment with different classes of genotoxins. Mutat. Res. 493:87-93.
Molinier, J., Oakeley, E.J., Niederhauser, O., Kovalchuk, I. and Hohn, B. (2005) Dynamic response of plant genome to ultraviolet radiation and other genotoxic stresses. Mutat. Res. 571:235-247.
Molinier, J., Ramos, C., Fritsch, O. and Hohn, B. (2004) CENTRIN2 modulates homologous recombination and nucleotide excision repair in Arabidopsis. Plant Cell 16:1633-1643.
Murray, M.G. and Thompson, W.F. (1980) Rapid isolation of high molecular weight plant DNA. Nucleic Acids Res. 8:4321-4325.
Neuteboom, L.W., Lindhout, B.I., Saman, I.L., Hooykaas, P.J.J. and Van der Zaal, B.J. (2006) Effects of different zinc finger transcription factors on genomic targets. Biochem. Biophys. Res. Commun. 339:263-270.
Norskov-Lauritsen, N., Ebbesen, P., Demant, E.J. and Christensen, J.M. (1990) Bleomycin-iron complexes and DNA radiation damage. Cancer Biochem. Biophys. 11:265-273.
Offringa, R., de Groot, M.J.A., Haagsman, H.J., Does, M.P., van den Elzen, P.J.M. and Hooykaas, P.J.J. (1990) Extrachromosomal Homologous Recombination and Gene Targeting in Plant-Cells After Agrobacterium Mediated Transformation. EMBO J. 9:3077-3084.
Park, K.S., Jang, Y.S., Lee, H. and Kim, J.S. (2005a) Phenotypic alteration and target gene identification using combinatorial libraries of zinc finger proteins in prokaryotic cells. J. Bacteriol. 187:5496-5499.
Park, K.S., Seol, W., Yang, H.Y., Lee, S.I., Kim, S.K., Kwon, R.J., Kim, E.J., Roh, Y.H., Seong, B.L. and Kim, J.S. (2005b) Identification and use of zinc finger transcription factors that increase production of recombinant proteins in yeast and mammalian cells. Biotechnol. Prog. 21:664-670.
Park, K.S., Lee, D.K., Lee, H., Lee, Y., Jang, Y.S., Kim, Y.H., Yang, H.Y., Lee, S.I., Seol, W. and Kim, J.S. (2003) Phenotypic alteration of eukaryotic cells using randomized libraries of artificial transcription factors. Nat. Biotechnol. 21:1208-1214. Paszkowski, J., Baur, M., Bogucki, A. and Potrykus, I. (1988) Gene Targeting in Plants. EMBO J. 7:4021-4026.
Sadowski, I., Ma, J., Triezenberg, S. and Ptashne, M. (1988) GAL4-VP16 is an unusually potent transcriptional activator. Nature 335:563-564.
Schuermann, D., Molinier, J., Fritsch, O. and Hohn, B. (2005) The dual nature of homologous recombination in plants. Trends Genet. 21:172-181.
Segal, D.J., Dreier, B., Beerli, R.R. and Barbas, C.F. (1999) Toward controlling gene expression at will: Selection and design of zinc finger domains recognizing each of the 5 '-GNN-3' DNA target sequences. Proc. Natl. Acad. Sci. U.S.A. 96:2758-2763. Swoboda, P., Gal, S., Hohn, B. and Puchta, H. (1994) Intrachromosomal homologous recombination in whole plants. EMBO J. 13:484-489.
Takeda, S., Tadele, Z., Hofmann, I., Probst, A.V., Angelis, K.J., Kaya, H., Araki, T., Mengiste, T., Mittelsten, S.O., Shibahara, K., Scheel, D. and Paszkowski, J. (2004) BRU1, a novel link between responses to DNA damage and epigenetic gene silencing in Arabidopsis. Genes Dev. 18:782-793.
Van Eenennaam, A.L., Li, G., Venkatramesh, M., Levering, C., Gong, X., Jamieson, A.C., Rebar, E.J., Shewmaker, C.K. and Case, C.C. (2004) Elevation of seed alpha-tocopherol levels using plant-based transcription factors targeted to an endogenous locus. Metab. Eng. 6:101-108.
Weigel, D., Ahn, J.H., Blazquez, M.A., Borevitz, J.O., Christensen, S.K., Fankhauser, C., Ferrandiz, C., Kardailsky, I., Malancharuvil, E.J., Neff, M.M., Nguyen, J.T., Sato, S., Wang, Z.Y., Xia, Y.J., Dixon, R.A., Harrison, M.J., Lamb, C.J., Yanofsky, M.F. and Chory, J. (2000) Activation tagging in Arabidopsis. Plant Physiol. 122:1003-1013.
Weijers, D., Franke-van Dijk, M., Vencken, R.J., Quint, A., Hooykaas, P.J.J. and Offringa, R. (2001) An Arabidopsis Minute-like phenotype caused by a semi-dominant mutation in a RIBOSOMAL PROTEIN S5 gene. Development 128:4289-4299.

## Claims

1. Method to generate mutants in plants, preferably *Arabidopsis,* by transforming said plants with pools of zinc finger artificial transcription factors (ZF-ATFs) and selecting a mutant from those transformed plant with a desired phenotype.

2. Method according to claim 1, wherein the desired phenotype is an increase frequency of homologous recombination.

3. Method according to claim 1, wherein the pools of ZF-ATFs comprises ZF-ATFs possessing three zinc fingers (3F).

4. Plant, transformed with a nucleotide sequence encoding the amino acid sequence as depicted in Fig. 5B, preferably wherein said nucleotide sequence is the sequence as depicted in Fig. 5A.

5. Plant according to claim 4, which is transformed with a nucleotide sequence encoding the amino acid sequence as depicted in Fig. 4B, preferably wherein said nucleotide sequence is the sequence as depicted in Fig. 4A.

6. Plant according to claim 4 or 5, wherein said plant is of the genus *Arabidopsis.*

7. Method to isolate genes which are involved in homologous recombination in plants, by
- producing mutants according to claim 1,
- selecting from such mutants one or more mutants with a desired phenotype,
- mapping the recognition sequences, which recognize the specific ZF-ATF with which said mutant was transformed,
- isolate the genes which comprise said recognition sequence or which are flanking said recognition sequence, and
- testing whether these genes are involved in homologous recombination.

8. Method to isolate genes which are involved in homologous recombination in plants, by
- producing mutants according to claim 1,
- selecting from such mutants one or more mutants with a desired phenotype,
- using plant material obtained from plants according to any of claims 4-6 for the identification of genes differently expressed as a result of the specific ZF-ATF
- testing whether these genes are involved in homologous recombination.


SEQUENCE LISTING

<110>  Universiteit Leiden

<120>  Plant homologous recombination mutants

<130>  P77813EP00

<140>  EP 06076281.2
<141>  2006-06-22

<160>  7

<170>  PatentIn version 3.3

<210>  1
<211>  17
<212>  DNA
<213>  Artificial

<220>
<223>  Primer pRF-uni-fw

<400>  1
gaagcgtaag gtcgagc                                                    17


<210>  2
<211>  17
<212>  DNA
<213>  Artificial

<220>
<223>  Primer pRF-2pol-rev

<400>  2
ctcgcgaatg catcgag                                                    17


<210>  3
<211>  22
<212>  DNA
<213>  Artificial

<220>
<223>  Primer M13R

<400>  3
caggaaacag ctatgaccat ga                                              22


<210>  4
<211>  864
<212>  DNA
<213>  Artificial

<220>
<223>  VP16-HRU gene


<220>

<221>  CDS
<222>  (1)..(861)
<223>  VP16-HRU gene

<400>  4
atg gcc gac tac aag gac gac gac gat aag cgg ccg ctt gag cct ccg        48
Met Ala Asp Tyr Lys Asp Asp Asp Asp Lys Arg Pro Leu Glu Pro Pro
1               5                   10                  15

aaa aag aag cgt aag gtc gag cta gca ggt acc gag ctt gcc ccc ccg        96
Lys Lys Lys Arg Lys Val Glu Leu Ala Gly Thr Glu Leu Ala Pro Pro
            20                  25                  30

acc gat gtc agc ctg ggg gac gag ctc cac tta gac ggc gag gac gtg       144
Thr Asp Val Ser Leu Gly Asp Glu Leu His Leu Asp Gly Glu Asp Val
        35                  40                  45

gcg atg gcg cat gcc gac gcg cta gac gat ttc gat ctg gac atg ttg       192
Ala Met Ala His Ala Asp Ala Leu Asp Asp Phe Asp Leu Asp Met Leu
    50                  55                  60

ggg gac ggg gat tcc ccg ggt ccg gga ttt acc ccc cac gac tcc gcc       240
Gly Asp Gly Asp Ser Pro Gly Pro Gly Phe Thr Pro His Asp Ser Ala
65                  70                  75                  80

ccc tac ggc gct ctg gat atg gcc gac ttc gag ttt gag cag atg ttt       288
Pro Tyr Gly Ala Leu Asp Met Ala Asp Phe Glu Phe Glu Gln Met Phe
                85                  90                  95

acc gat gcc ctt gga att gac gag tac ggt ggt cta gaa tcg cga gaa       336
Thr Asp Ala Leu Gly Ile Asp Glu Tyr Gly Gly Leu Glu Ser Arg Glu
            100                 105                 110

ttc cac gtg ctc gag gtc gag gcc cag gcg gcc ctc gaa ccc acc ggc       384
Phe His Val Leu Glu Val Glu Ala Gln Ala Ala Leu Glu Pro Thr Gly
        115                 120                 125

gag aaa ccg tat gcg tgc ccc gaa tgc ggg aaa tcc ttt tcg acc agc       432
Glu Lys Pro Tyr Ala Cys Pro Glu Cys Gly Lys Ser Phe Ser Thr Ser
    130                 135                 140

ggt gaa ctc gtg cgt cac cag cgt acg cat acc ggc gag aag ccc tac       480
Gly Glu Leu Val Arg His Gln Arg Thr His Thr Gly Glu Lys Pro Tyr
145                 150                 155                 160

gct tgc ccg gag tgt ggc aag tca ttc tcc agg agc gac aat ttg gtt       528
Ala Cys Pro Glu Cys Gly Lys Ser Phe Ser Arg Ser Asp Asn Leu Val
                165                 170                 175

agg cat caa cgc aca cat acc ggc gaa aag ccg tat gcg tgc ccc gaa       576
Arg His Gln Arg Thr His Thr Gly Glu Lys Pro Tyr Ala Cys Pro Glu
            180                 185                 190

tgt ggg aag tcc ttt tct cgt agc gac gag ctt gtt cgc cac caa agg       624
Cys Gly Lys Ser Phe Ser Arg Ser Asp Glu Leu Val Arg His Gln Arg
        195                 200                 205

acc cat acc ggc gag ctg ggt ggc ggt tcc gga gaa aag ccc ggg aag       672
Thr His Thr Gly Glu Leu Gly Gly Gly Ser Gly Glu Lys Pro Gly Lys
    210                 215                 220

aaa acc tcg ggc cag gcc ggc caa cta gtg aat tcg aag atc tcg atg 720
Lys Thr Ser Gly Gln Ala Gly Gln Leu Val Asn Ser Lys Ile Ser Met
225                 230                 235                 240

cat tcg cga ggc gcc gtc gac ggg cgg ccg ctc gag gtt atg gca gaa 768
His Ser Arg Gly Ala Val Asp Gly Arg Pro Leu Glu Val Met Ala Glu
                245                 250                 255

gtt cgg ccc ata gcc cga tcc att aaa acg gct cac gac gat gcg cga 816
Val Arg Pro Ile Ala Arg Ser Ile Lys Thr Ala His Asp Asp Ala Arg
            260                 265                 270

gcg gaa tta atg tcg gcg gac aga cct cga tca acg cgc ggt cta tga 864
Ala Glu Leu Met Ser Ala Asp Arg Pro Arg Ser Thr Arg Gly Leu
        275                 280                 285

<210> 5
<211> 287
<212> PRT
<213> Artificial

<220>
<223> Synthetic Construct

<400> 5

Met Ala Asp Tyr Lys Asp Asp Asp Asp Lys Arg Pro Leu Glu Pro Pro
1               5                   10                  15

Lys Lys Lys Arg Lys Val Glu Leu Ala Gly Thr Glu Leu Ala Pro Pro
            20                  25                  30

Thr Asp Val Ser Leu Gly Asp Glu Leu His Leu Asp Gly Glu Asp Val
        35                  40                  45

Ala Met Ala His Ala Asp Ala Leu Asp Asp Phe Asp Leu Asp Met Leu
    50                  55                  60

Gly Asp Gly Asp Ser Pro Gly Pro Gly Phe Thr Pro His Asp Ser Ala
65                  70                  75                  80

Pro Tyr Gly Ala Leu Asp Met Ala Asp Phe Glu Phe Glu Gln Met Phe
                85                  90                  95

Thr Asp Ala Leu Gly Ile Asp Glu Tyr Gly Gly Leu Glu Ser Arg Glu
            100                 105                 110

Phe His Val Leu Glu Val Glu Ala Gln Ala Ala Leu Glu Pro Thr Gly
        115                 120                 125

Glu Lys Pro Tyr Ala Cys Pro Glu Cys Gly Lys Ser Phe Ser Thr Ser
    130                 135                 140

Gly Glu Leu Val Arg His Gln Arg Thr His Thr Gly Glu Lys Pro Tyr
145                 150                 155                 160

Ala Cys Pro Glu Cys Gly Lys Ser Phe Ser Arg Ser Asp Asn Leu Val
                165                 170                 175

Arg His Gln Arg Thr His Thr Gly Glu Lys Pro Tyr Ala Cys Pro Glu
            180                 185                 190

Cys Gly Lys Ser Phe Ser Arg Ser Asp Glu Leu Val Arg His Gln Arg
        195                 200                 205

Thr His Thr Gly Glu Leu Gly Gly Gly Ser Gly Glu Lys Pro Gly Lys
    210                 215                 220

Lys Thr Ser Gly Gln Ala Gly Gln Leu Val Asn Ser Lys Ile Ser Met
225                 230                 235                 240

His Ser Arg Gly Ala Val Asp Gly Arg Pro Leu Glu Val Met Ala Glu
                245                 250                 255

Val Arg Pro Ile Ala Arg Ser Ile Lys Thr Ala His Asp Asp Ala Arg
            260                 265                 270

Ala Glu Leu Met Ser Ala Asp Arg Pro Arg Ser Thr Arg Gly Leu
        275                 280                 285

<210>  6
<211>  258
<212>  DNA
<213>  Artificial

<220>
<223>   Zinc finger coding sequence

<220>
<221>  CDS
<222>  (1)..(258)
<223>  3 Zinc finger sequence

<400>  6
acc ggc gag aaa ccg tat gcg tgc ccc gaa tgc ggg aaa tcc ttt tcg       48
Thr Gly Glu Lys Pro Tyr Ala Cys Pro Glu Cys Gly Lys Ser Phe Ser
1               5                   10                  15

acc agc ggt gaa ctc gtg cgt cac cag cgt acg cat acc ggc gag aag       96
Thr Ser Gly Glu Leu Val Arg His Gln Arg Thr His Thr Gly Glu Lys
            20                  25                  30

ccc tac gct tgc ccg gag tgt ggc aag tca ttc tcc agg agc gac aat          144
Pro Tyr Ala Cys Pro Glu Cys Gly Lys Ser Phe Ser Arg Ser Asp Asn
        35                  40                  45

ttg gtt agg cat caa cgc aca cat acc ggc gaa aag ccg tat gcg tgc          192
Leu Val Arg His Gln Arg Thr His Thr Gly Glu Lys Pro Tyr Ala Cys
    50                  55                  60

ccc gaa tgt ggg aag tcc ttt tct cgt agc gac gag ctt gtt cgc cac          240
Pro Glu Cys Gly Lys Ser Phe Ser Arg Ser Asp Glu Leu Val Arg His
65                  70                  75                  80

caa agg acc cat acc ggc                                                  258
Gln Arg Thr His Thr Gly
                85

<210>  7
<211>  86
<212>  PRT
<213>  Artificial

<220>
<223>  Synthetic Construct

<400>  7

Thr Gly Glu Lys Pro Tyr Ala Cys Pro Glu Cys Gly Lys Ser Phe Ser
1               5                   10                  15

Thr Ser Gly Glu Leu Val Arg His Gln Arg Thr His Thr Gly Glu Lys
            20                  25                  30

Pro Tyr Ala Cys Pro Glu Cys Gly Lys Ser Phe Ser Arg Ser Asp Asn
        35                  40                  45

Leu Val Arg His Gln Arg Thr His Thr Gly Glu Lys Pro Tyr Ala Cys
    50                  55                  60

Pro Glu Cys Gly Lys Ser Phe Ser Arg Ser Asp Glu Leu Val Arg His
65                  70                  75                  80

Gln Arg Thr His Thr Gly
                85